# EUROPÄISCHE PATENTSCHRIFT

(11) **EP 2 440 642 B1**
(45) Veröffentlichungstag und Bekanntmachung des Hinweises auf die Patenterteilung: **25.10.2017**
(21) Anmeldenummer: 10713946.1
(22) Anmeldetag: 15.04.2010
(51) Int. Cl.: C11D 3/30, C11D 3/00, C11D 3/50, A61Q 15/00, A61K 8/41, A61L 9/01

(54) **VERFAHREN ZUR VERMINDERUNG VON SCHLECHTGERÜCHEN**
METHOD FOR REDUCING MALODORS
PROCÉDÉ DE RÉDUCTION DE MAUVAISES ODEURS

(30) Priorität: 09.06.2009 DE 102009026856
(43) Veröffentlichungstag der Anmeldung: 18.04.2012
(73) Patentinhaber: Henkel AG & Co. KGaA, 40589 Düsseldorf (DE)
(72) Erfinder: HUCHEL, Ursula, 50670 Köln (DE); BAUER, Andreas, 41564 Kaarst (DE); GERKE, Thomas, 40627 Düsseldorf (DE)
(74) Vertreter: Viering, Jentschura & Partner mbB Patent- und Rechtsanwälte
(86) Internationale Anmeldenummer: PCT/EP2010/054917
(87) Internationale Veröffentlichungsnummer: WO 2010/142479

(56) Entgegenhaltungen:
- EP-A1- 1 787 689
- JP-A- 2007 209 464
- JP-A- 2007 291 540
- JP-A- 2007 300 963
- US-A- 3 755 085

## Beschreibung

Die vorliegende Erfindung betrifft ein Verfahren zur Verminderung von Fehlgerüchen. Ferner betrifft es die Verwendung von Amino-1,3-propandiol und/oder substituiertem 2-Amino-1,3-propandiol in Wasch-, Reinigungs- oder Pflegemitteln zum Abbau von Fehlgerüchen, hervorgerufen durch die Anwesenheit von Aldehyden und/oder Ketonen. EP1787689 A1, JP2007209464 A, JP2007291540 A and JP2007300963 A offenbaren ein Verfahren zur Reduzierung von Fehlgerüchen, verursacht durch die Anwesenheit von Aldehyden. wobei ein Pflegemittel enthaltend 2-Amino-1,3-propanol und/oder substituiertes 2-Amino-1,3-propanol eingesetzt wird.

US3755085 A offenbart Wasch- oder Reinigungsmittel enthaltend 2-Amino-1,3-propanol und/oder substituiertes 2-Amino-1,3-propanol als Chlorfänger. Ein wichtiges Bedürfnis, welches insbesondere der Anwendung von Wasch-, Reinigungs- oder Pflegemitteln zu Grunde liegt, besteht in der Beseitigung oder zumindest in der Verminderung von Schlechtgerüchen (d.h. Fehlgerüchen) bzw. unerwünschten Gerüchen. Schlecht- bzw.

Fehlgerüche gehen von bestimmten geruchsaktiven Verbindungen aus, wie z.B. bestimmten Ketonen oder Aldehyden, z.B. 2,4-Decadienal, insbesondere kurzkettigen Aldehyden.

Aufgabe der vorliegenden Erfindung war es, dem Verbraucher eine Möglichkeit zu geben, eine Verminderung von Fehlgerüchen, die auf Aldehyde und/oder Ketone zurückzuführen sind, herbeizuführen.

Gelöst wird diese Aufgabe durch den Gegenstand der Erfindung, nämlich ein Verfahren zum Abbau von Fehlgerüchen, hervorgerufen durch die Anwesenheit von Aldehyden und/oder Ketonen, insbesondere kurzkettigen Aldehyden, welches durch die Zugabe von 2-Amino-1,3-propandiol und/oder substituiertem 2-Amino-1,3-propandiol gemäß der Formel (I) 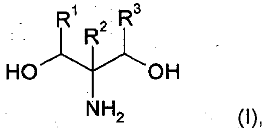 wobei in dieser Formel die Reste R¹, R² sowie R³, jeweils unabhängig voneinander, für Wasserstoff oder Kohlenwasserstoffreste stehen, gekennzeichnet ist. R¹ und R³ stehen insbesondere, jeweils unabhängig voneinander, für C₁₋₆-Alkylreste, vorzugsweise C₁₋₃-Alkylreste, oder für Wasserstoff. Besonders bevorzugte Reste R² sind neben Wasserstoff insbesondere Methyl-, Ethyl-, und Hydroxymethylreste. In einer bevorzugten Ausführungsform bedeuten R¹, R² und R³ jeweils Wasserstoff.

Der Begriff "Zugabe" ist hier im weitesten Sinne zu verstehen, d.h. es wird 2-Amino-1,3-propandiol und/oder substituiertem 2-Amino-1,3-propandiol eingesetzt, um die genannten Fehlgerüche bzw. unerwünschte Gerüche zu vermindern. Immer wenn ein Fehlgeruch vorliegt, muss es ein Objekt geben, von dem dieser Fehlgeruch ausgeht bzw. einen Raum bzw. ein System, in welchem der Fehlgeruch wahrnehmbar ist. Zu diesem Objekt bzw. in diesen Raum bzw. in das System kann erfindungsgemäß das 2-Amino-1,3-propandiol und/oder substituiertes 2-Amino-1,3-propandiol zugegeben werden.
Das 2-Amino-1,3-propandiol und/oder substituiertes 2-Amino-1,3-propandiol kann z.B. in die Raumluft gesprüht werden, es kann in einen Behälter mit Wasser oder einer Waschlauge gegeben werden, es kann direkt auf jedes abgegrenzte Objekt, also z.B. auch ein verschwitztes Hemd bzw. eine Vielzahl solcher Objekte, wie z.B. ein Waschgut bestehend aus vielen verschmutzten Hemden oder ähnlichem, gegeben werden.

Es konnte überraschend gefunden werden, dass das erfindungsgemäße Verfahren eine deutliche Verminderung von Schlecht- bzw. Fehlgerüchen bzw. unerwünschten Gerüchen, die auf Ketone und/oder Aldehyde insbesondere kurzkettige Aldehyde zurückzuführen sind, ermöglicht.

In einer bevorzugten Ausführungsform der Erfindung wird das 2-Amino-1,3-propandiol und/oder substituierte 2-Amino-1,3-propandiol gemäß Formel (I) zusammen mit Riechstoffen eingesetzt. Geeignete Riechstoffe werden weiter unten beschrieben.

Es ist besonders bevorzugt, dass die Zugabe des 2-Amino-1,3-propandiols und/oder des substituierten 2-Amino-1,3-propandiols gemäß Formel (I) durch Sprühen erfolgt. Dies entspricht einer bevorzugten Ausführungsform der Erfindung. Wenn die Zugabe mittels Spraydose (Druckgasdose, Druckgaspackung, Aerosolpackung) oder mechanisch zu bedienendem Pumpzerstäuber (Pumpspray) erfolgt, unter Bildung eines Sprühnebels, Schaums, Paste oder Flüssigkeitsstrahls, so liegt wiederum eine bevorzugte Ausführungsform der Erfindung vor.

Gemäß einer weiter bevorzugten Ausführungsform der Erfindung erfolgt die Zugabe des 2-Amino-1,3-pröpandiols und/oder des substituierten 2-Amino-1,3-propandiol gemäß Formel (I) im Rahmen eines Wasch- oder Reinigungsprozesses. Dabei wird das 2-Amino-1,3-propandiol und/oder substituierte 2-Amino-1,3-propandiol insbesondere als Bestandteil eines, vorzugsweise riechstoffhaltigen, Wasch-, Reinigungs- oder Pflegemittels eingesetzt. Hierbei erfolgt eine besonders gute Verminderung des Schlechtgeruches.

In einem Verfahren gemäß der Erfindung kann ein Wasch-, Reinigungs- oder Pflegemittel eingesetzt werden, welches 2-Amino-1,3-propandiol und/oder substituiertes 2-Amino-1,3-propandiol gemäß der Formel (I) 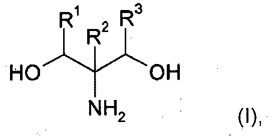 wobei in dieser Formel die Reste R¹, R² sowie R³, jeweils unabhängig voneinander, für Wasserstoff oder Kohlenwasserstoffreste stehen, bevorzugt in Mengen von 0,001 Gew.-% bis 5 Gew.-%, vorteilhafterweise 0,005 Gew.-% bis 3 Gew.-%, vorzugsweise 0,01 bis 2 Gew.-%, insbesondere 0,1 bis 1 Gew.-%, enthält, Gew.-% bezogen auf das gesamte Mittel. R¹ und R³ stehen insbesondere, jeweils unabhängig voneinander, für C₁₋₆-Alkylreste, vorzugsweise C₁₋₃-Alkylreste, oder für Wasserstoff. Besonders bevorzugte Reste R² sind neben Wasserstoff insbesondere Methyl-, Ethyl-, und Hydroxymethylreste. In einer bevorzugten Ausführungsform bedeuten R¹, R² und R³ jeweils Wasserstoff.

Es konnte überraschend gefunden werden, dass die Anwendung eines Wasch-, Reinigungs- oder Pflegemittels eine deutliche Verminderung von Schlechtgerüchen bzw. unerwünschten Gerüchen, die auf Ketone und/oder Aldehyde, insbesondere kurzkettige Aldehyde zurückzuführen sind, ermöglicht. Außerdem konnte beim Einsatz der Mittel ein verbessertes Dufterlebnis erreicht werden, insbesondere im Zusammehang mit der Textilbehandlung.

Die Mittel eignen sich prinzipiell gleichermaßen zur Textilbehandlung wie zur Reinigung harter Oberflächen, z.B. Fußboden, sowie für den kosmetischen Einsatz sowie für den Air-Care-Bereich bzw. die Raumluftverbesserung bzw. als Geruchsbekämpfungsmittel

Das Mittel kann in fester Form vorliegen, vorzugsweise in Pulverform oder auch in Granulatform oder in Gestalt von Pressformkörpern, z.B. Tabletten. Die Mittel in fester Form sind insbesondere für die Anwendung in wässrigen Systemen vorgesehen, z. B. für den Einsatz in einer Wasch- oder Reinigungslauge. Es ist aber auch möglich, dass das Mittel in flüssiger Form vorliegt, vorzugsweise in Gelform. Flüssige Mittel können auch direkt angewendet werden, z.B. durch Auftragen auf ein übelriechendes Objekt, ggf. können sie vorher verdünnt werden. Ebenso ist die die Anwendung in wässrigen Systemen möglich.

Neben dem 2-Amino-1,3-propandiol und/oder substituiertes 2-Amino-1,3-propandiol kann das Mittel weitere geeignete Inhaltsstoffe umfassen, insbesondere aber Tenside. Wenn das Mittel zumindest 5 Gew.-%, vorzugsweise zumindest 8 Gew.-%, insbesondere zumindest 10 Gew.-% Tensid enthält, insbesondere Aniontensid und/oder Niotensid, so liegt eine bevorzugte Ausführungsform vor. Eine sinnvolle Obergrenze für Tensid kann z.B. bei 40 Gew.-% oder 30 Gew.-% oder 20 Gew.-% liegen, Gew.-% jeweils bezogen auf das gesamte Mittel. So kann eine besonders gute, erfindungsgemäß angestrebte Verminderung von Fehlgerüchen bzw. unerwünschten Gerüchen bewirkt werden. Tenside werden weiter unten noch genauer beschrieben.

Nach einer weiteren Ausführungsform sind in dem Mittel Duftstoff(e) enthalten, insbesondere ausgewählt aus der Gruppe umfassend Duftstoffe natürlichen oder synthetischen Ursprungs, bevorzugt leichter flüchtige Duftstoffe, höhersiedende Duftstoffe, feste Duftstoffe und/oder haftfeste Duftstoffe. Haftfeste Riechstoffe, die im Rahmen der vorliegenden Erfindung mit Vorteil einsetzbar sind, sind z.B. etherische Öle wie Angelikawurzelöl, Anisöl, Arnikablütenöl, Basilikumöl, Bayöl, Bergamottöl, Champacablütehöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaiva-balsamöl, Korianderöl, Krauseminzeöl, Kümmelöl, Kuminöl, Lavendelöl, Lemongrasöl, Limetteöl, Mandarinenöl, Melissenöl, Moschuskömeröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfeffer-minzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl. Aber auch höhersiedende bzw. feste Riechstoffe natürlichen oder synthetischen Ursprungs können im Rahmen der vorliegenden Erfindung als haftfeste Riechstoffe bzw. Riechstoffgemische, also Duftstoffe eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbon-säuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methyl-anthranilsäuremethylester, p-Methylacetophenon, Methylchavikol, p-Methylchinolin, Methyl-β-Naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylalkohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undecalacton, Vanillin,

Veratrumaldehyd, Zimtaldehyd, Zimatalkohol, Zimtsäure, Zimtsäureethylester, Zimtsäurebenzylester. Zu den leichter flüchtigen Duftstoffen zählen insbesondere die niedriger siedenden Riechstoffe natürlichen oder synthetischen Ursprung, die allein oder in Mischungen eingesetzt werden können. Beispiele für leichter flüchtige Duftstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Der Gehalt an optionalen Duftstoffen in dem Mittel beträgt vorzugsweise 0,001 Gew.-% bis 10 Gew.-%, vorteilhafterweise 0,01 bis 5 Gew.-% und insbesondere 0,1 Gew.-% bis 3 Gew.-%, Gew.-% bezogen auf das gesamte Mittel. Der kombinierte Einsatz von 2-Amino-1,3-propandiol und/oder substituiertes 2-Amino-1,3-propandiol mit Duftstoffen, insbesondere in Kombination mit Tensiden ermöglicht eine ganz besonders gute, erfindungsgemäß angestrebte Verminderung von Fehlgerüchen.

Zur beschleunigten oder verzögerten Freisetzung von Duftstoffen können alle im Stand der Technik bekannten Verfahren angewendet werden, soweit sie dem Fachmann als geeignet erscheinen. Gemäß einer Ausführungsform umfasst das Mittel geträgerten und/oder verkapselten Duftstoff.

Ein Wasch-, Reinigungs- oder Pflegemittels zum Reinigen und/oder Konditionieren von textilen Flächengebilden kann, insbesondere in einer automatischen Waschmaschine, vorzugsweise bei Temperaturen nicht über 60 °C, insbesondere nicht über 40 °C verwendet werden.

Ebenso ist ein weiterer Gegenstand der Erfindung die Verwendung eines erfindungsgemäßen Wasch-, Reinigungs- oder Pflegemittels zum Reinigen harter Oberflächen.

Ein weiterer Gegenstand der Erfindung liegt in der Verwendung von Amino-1,3-propandiol und/oder substituiertem 2-Amino-1,3-propandiol gemäß der Formel (I) in (vorzugsweise riechstoffhaltigen) Wasch-, Reinigungs- oder Pflegemitteln zum Abbau von Fehlgerüchen, hervorgerufen durch die Anwesenheit von Aldehyden und/oder Ketonen, insbesondere kurzkettigen Aldehyden.

Bei dem Wasch-, Reinigungs- oder Pflegemittel kann es sich insbesondere um ein Textilbehandlungsmittel in Form eines Textilwaschmittels, Weichspülers, weichmachenden Waschmittels oder Waschhilfsmittels handeln. Ebenso kann es sich z.B. um ein Reinigungsmittel für harte Oberflächen handeln, wie vorzugsweise um ein Geschirrspülmittel, insbesondere um ein maschinelles Geschirrspülmittel. Ebenso kann es sich um Reinigungsmittel wie z.B. Haushaltsreiniger, Allzweckreiniger, Fensterreiniger, Fußbodenreiniger usw. handeln. Vorzugsweise kann es sich um ein
Produkt zur Reinigung von WC-Becken und Urinalen handeln, vorteilhafterweise um einen Spülreiniger zum Einhängen in das WC-Becken oder den Spülkasten, insbesondere um einen sogenannten WC-Stein. Im Sinne des Pflegemittels handelt es sich bevorzugt um Kosmetika, wie z.B. Haarshampoos, Deos usw., welche für die Körperpflege und/oder -reinigung einsetzbar sind. Im Sinne des Pflegemittels kann es sich ferner auch um Air-Care-Produkte und Raumluftverbesserer handeln.

Gemäß einer Ausführungsform handelt es sich bei dem Mittel um ein Textilbehandlungsmittel, ein Bügelhilfsmittel, ein Reinigungstuch, ein Textilwaschmittel, einen Weichspüler, ein Reinigungsmittel, insbesondere für harte und/oder weiche Oberflächen, einen Haushaltsreiniger, ein Pflegemittel, ein Waschpflegemittel, ein Raumbeduftungsmittel, einen Luftverbesserer, ein Konditioniermittel, ein Färbemittel, einen Weichspüler, ein Konditioniersubstrat, ein Putzmittel, ein kosmetisches Mittel, ein Bleichmittel, ein Entkalkungsmittel, ein Autopflegemittel, Fußbodenpflegemittel, Herdpflegemittel, Lederpflegemittel, Möbelpflegemittel, ein Scheuermittel, ein Desinfektionsmittel, ein Beduftungsmittel, ein Schimmelentfernungsmittel und/oder ein Vorprodukt der vorgenannten Mittel. Es ist ein Vorteil, dass die Mittel sehr lagerstabil sind.

Nach einer Ausführungsform weist das Wasch-, Pflege- oder Reinigungsmittel, wenigstens eine, vorzugsweise mehrere, aktive Komponenten, insbesondere wasch-, pflege-, reinigungsaktive und/oder kosmetische Komponenten auf, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositiorismittel, Antistatika, Buildersubstanzen, Bleichmittel, Bleichaktivatoren, Bleichstabilisatoren, Bleichkatalysatoren, Bügelhilfsmittel, Cobuilder, Duftstoffe, Einlaufverhinderer, Elektrolyte, Enzyme, Farbschutzstoffe, Färbemittel, Farbstoffe, Farbübertragungsinhibitoren,

Fluoreszenzmittel, Fungizide, Germizide, geruchskomplexierende Substanzen, Hilfsmittel, Hydrotrope, Klarspüler, Komplexbildner, Konservierungsmittel, Korrosionsinhibitoren, wassermischbare organische Lösungsmittel, optische Aufheller, Parfüme, Parfümträger, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silberschutzmittel, Silikonöle, Soil release-Wirkstoffe, UV-Schutz-Substanzen, Viskositätsregulatoren, Verdickungsmittel, Verfärbungsinhibitoren, Vergrauungsinhibitoren, Vitamine und/oder Weichspüler. Im Sinne dieser Erfindung beziehen sich Angaben für das Mittel in Gew.-%, wenn nicht anders angegeben, auf das Gesamtgewicht des Mittels.

Die Mengen der einzelnen Inhaltsstoffe in den Mitteln orientieren sich jeweils am Einsatzzweck der betreffenden Mittel und der Fachmann ist mit den Größenordnungen der einzusetzenden Mengen der Inhaltsstoffe vertraut oder kann diese der zugehörigen Fachliteratur entnehmen. Je nach Einsatzzweck der Mittel wird man z.B. den Tensidgehalt höher öder niedriger wählen. Üblicherweise kann z. B. der Tensidgehalt beispielsweise von Waschmitteln zwischen 10 und 50 Gew.-%, vorzugsweise zwischen 12,5 und 30 Gew.-% und insbesondere zwischen 15 und 25 Gew.-% betragen, während Reinigungsmittel für das maschinelle Geschirrspülen z.B. zwischen 0,1 und 10 Gew.-%, vorzugsweise zwischen 0,5 und 7,5 Gew.-% und insbesondere zwischen 1 und 5 Gew.-% Tenside enthalten können.

Die Mittel können Tenside enthalten, wobei bevorzugt anionische Tenside, nichtionische Tenside und deren Gemische, aber auch kationische Tenside in Frage kommen. Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar. Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen,
sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X⁻ ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X⁻ für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxysubstituierte Alkyl-Reste verfügen, sind wenig reaktiv und werden z.B. mit Dimethylsulfat quaterniert. In Frage kommende QAV sind z.B. Benzalkoniumchlorid (N Alkyl-N,N dimethylbenzylammoniumchlorid), Benzalkon B (m,p-Dichlorbenzyl-dimethyl-C₁₂-alkylammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N [2-[2-[p-(1,1,3,3-tetramethylbutyl)phenoxy]-ethoxy]-ethyl]-benzylammoniumchlorid), Dialkyldimethylammoniumchloride wie Di-n-decyl-dimethyl-ammoniumchlorid, Didecyldimethylammonium-bromid, Dioctyl-dimethyl-ammoniumchlorid, 1-Cetylpyridiniumchlorid und Thiazolinjodid sowie deren Mischungen. Bevorzugte QAV sind die Benzalkoniumchloride mit C₈-C₂₂-Alkylresten, insbesondere C₁₂-C₁₄-Alkyl-benzyl-dimethylammoniumchlorid.

Unter Esterquats sollen hier vorzugsweise Verbindungen der allgemeinen Formel IV, 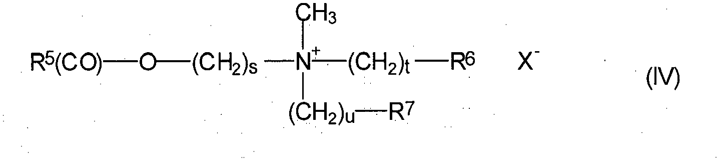 verstanden werden, in der R⁵ für einen Alkyl- oder Alkenylrest mit 12 bis 22 Kohlenstoffatomen und 0, 1, 2 oder 3 Doppelbindungen, R⁶ und R⁷ unabhängig voneinander für H, OH oder O(CO)R⁵, s, t und u jeweils unabhängig voneinander für den Wert 1, 2 oder 3 und X⁻ für ein Anion, insbesondere Halogenid, Methosulfat, Methophosphat oder Phosphat sowie Mischungen aus diesen, steht.

Bevorzugt sind Verbindungen, die für R⁶ die Gruppe O(CO)R⁵ und für R⁵ einen Alkylrest mit 16 bis 18 Kohlenstoffatomen enthalten. Besonders bevorzugt sind Verbindungen, bei denen R⁷ zudem für OH steht. Beispiele für Verbindungen der Formel (IV) sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Werden quarternierte Verbindungen der Formel (IV) eingesetzt, die ungesättigte Gruppen aufweisen, sind die Acylgruppen bevorzugt, deren korrespondierende Fettsäuren eine Jodzahl zwischen 5 und 80, vorzugsweise zwischen 10 und 60 und insbesondere zwischen 15 und 45 aufweisen und/oder die ein cis/trans-Isomerenverhältnis (in Mol-%) von größer als 30 : 70, vorzugsweise größer als 50 : 50 und insbesondere größer als 70 : 30 haben. Handelsübliche Beispiele sind die von der Firma Stepan unter dem Warenzeichen Stepantex® vertriebenen Methylhydroxyalkyldialkoyloxyalkylammoniummethosulfate oder die unter dem Handelsnamen Dehyquart® bekannten Produkte der Firma Cognis Deutschland GmbH beziehungsweise die unter der Bezeichnung Rewoquat® bekannten Produkte des Herstellers Goldschmidt-Witco.

Tenside sind gewünschtenfalls in den Mitteln in Mengenanteilen von vorzugsweise 5 Ges.-% bis 50 Gew.-%, insbesondere von 8 Gew.-% bis 30 Gew.-%, enthalten. Insbesondere in Wäschenachbehandlungsmitteln werden vorzugsweise bis zu 30 Gew.-%, insbesondere 5 Gew.-% bis 15 Gew.-% Tenside, unter diesen bevorzugt wenigstens anteilsweise Kationtenside, eingesetzt.

Ein zuvor genanntes Mittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphansäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättiger Carbonsäuren liegt im allgemeinen zwischen 5 000 und 200 000, die der Copolymeren zwischen 2 000 und 200 000, vorzugsweise 50 000 bis 120 000, jeweils bezogen auf freie Säure. Ein besonders bevorzugtes Acrylsäure-Maleinsäure-Copolymer weist eine relative Molekülmasse von 50 000 bis 100 000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50. Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/ oder ein Vinylalkohol-Derivat oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäure, insbesondere von (Meth)Acrylsäure ab. Das zweite saure Monomer beziehungsweise dessen Salz kann ein Derivat einer C₄-C₈-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, z.B. von C₁-C₄-Carbonsäuren, mit Vinylalkohol darstellen. Bevorzugte Polymere enthalten dabei 60 Gew.-% bis 95 Gew.-%, insbesondere 70 Gew.-% bis 90 Gew.-% (Meth)Acrylsäure bzw. (Meth)Acrylat, besonders bevorzugt Acrylsäure bzw. Acrylat, und Maleinsäure bzw. Maleinat sowie 5 Gew.-% bis 40 Gew.-%, vorzugsweise 10 Gew.-% bis 30 Gew.-% Vinylalkohol und/oder Vinylacetat. Ganz besonders bevorzugt sind dabei Polymere, in denen das Gewichtsverhältnis von (Meth)Acrylsäure beziehungsweise (Meth)Acrylat zu Maleinsäure beziehungsweise Maleinat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer beziehungsweise dessen Salz kann auch ein Derivat einer Allylsulfonsäure sein, die in 2-Stellung mit einem Alkylrest, vorzugsweise mit einem C₁-C₄-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.-% (Meth)Acrylsäure beziehungsweise (Meth)Acrylat, besonders bevorzugt Acrylsäure beziehungsweise Acrylat, 10 Gew.-% bis 30 Gew.-%, vorzugsweise 15 Gew.-% bis 25 Gew.-% Methallylsulfonsäure bzw. Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei z.B. ein Mono-, Di-, Oligo- oder Polysaccharid sein, wobei Mono-, Di- oder Oligosaccharide bevorzugt sind. Besonders bevorzugt ist Saccharose. Durch den Einsatz des dritten Monomers werden vermutlich Sollbruchstellen in das Polymer eingebaut, die für die gute biologische Abbaubarkeit des Polymers verantwortlich sind. Diese Terpolymere weisen im Allgemeinen eine relative Molekülmasse zwischen 1 000 und 200 000, vorzugsweise zwischen 200 und 50 000 und insbesondere zwischen 3 000 und 10 000 auf. Weitere bevorzugte Copolymere sind solche, die als Monomere Acrolein und Acrylsäure/Acrylsäuresalze beziehungsweise Vinylacetat aufweisen. Die organischen Buildersubstanzen können, insbesondere zur Herstellung flüssiger Mittel, in Form wäßriger Lösungen, vorzugsweise in Form 30- bis 50-gewichtsprozentiger wäßriger Lösungen eingesetzt werden. Alle genannten Säuren werden in der Regel in Form ihrer wasserlöslichen Salze, insbesondere ihre Alkalisalze, eingesetzt.

Organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, Mitteln eingesetzt. Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien werden insbesondere kristalline oder amorphe Alkalialumosilikate, in Mengen von bis zu 50 Gew.-%, vorzugsweise nicht über 40 Gew.-% und in flüssigen Mitteln insbesondere von 1 Gew.-% bis 5 Gew.-%, eingesetzt. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm.

Geeignete Substitute beziehungsweise Teilsubstitute für das genannte Alumosilikat sind kristalline Alkalisilikate, die allein oder im Gemisch mit amorphen Silikaten vorliegen können. Die in den Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl β- als auch δ-Natriumdisilikate (Na₂Si₂O₅ · y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in den genannten Mitteln eingesetzt werden. In einer Ausführungsform der Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren Ausführungsform der Mittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie

Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind in den Mitteln vorzugsweise in Mengen bis zu 60 Gew.-%, insbesondere von 5 Gew.-% bis 40 Gew.-%, enthalten. Wäschenachbehandlungsmittel, wie z.B. Weichspüler, sind vorzugsweise frei von anorganischem Builder.

Als geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder insbesondere in flüssigen Mitteln Wasserstoffperoxid in Form wäßriger Lösungen, die 3 Gew.-% bis 10 Gew.-% Wasserstoffperoxid enthalten, eingesetzt. Falls ein Mittel Bleichmittel, wie vorzugsweise Persauerstoffverbindungen, enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie z.B. von Phosphonaten, Boraten bzw. Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS); Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol beziehungsweise deren Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, z.B. N-Benzoylcaprolactam. Hydrophil substituierte Acylacetale und Acyllactame werden ebenfalls bevorzugt eingesetzt. Auch Kombinationen konventioneller Bleichaktivatoren können eingesetzt werden. Derartige Bleichaktivatoren können im üblichen Mengenbereich, vorzugsweise in Mengen von 1 Gew.-% bis 10 Gew.-%, insbesondere 2 Gew.-% bis 8 Gew.-%, bezogen auf gesamtes Mittel, enthalten sein.

Zusätzlich zu den oben aufgeführten konventionellen Bleichaktivatoren oder an deren Stelle können auch Sulfonimine und/oder bleichverstärkende Übergangsmetallsalze beziehungsweise Übergangsmetallkomplexe als sogenannte Bleichkatalysatoren enthalten sein. Zu den in Frage kommenden Übergangsmetallverbindungen gehören insbesondere Mangan-, Eisen-, Cobalt-, Ruthenium- oder Molybdän-Salenkomplexe und deren N-Analogverbindungen, Mangan-, Eisen-, Cobalt-, Ruthenium- oder Molybdän-Carbonylkomplexe, Mangan-, Eisen-, Cobalt-, Ruthenium-, Molybdän-, Titan-, Vanadium- und Kupfer-Komplexe mit stickstoffhaltigen Tripod-Liganden, Cobalt-, Eisen-, Kupfer- und Ruthenium-Amminkomplexe. Kombinationen aus Bleichaktivatoren und Übergangsmetall-Bleichkatalysatoren können ebenfalls eingesetzt werden. Bleichverstärkende Übergangsmetallkomplexe, insbesondere mit den Zentralatomen Mn, Fe, Co, Cu, Mo, V, Ti und/oder Ru, können in üblichen Mengen, vorzugsweise in einer Menge bis zu 1 Gew.-%, insbesondere von 0,0025 Gew.-% bis 0,25 Gew.-% und besonders bevorzugt von 0,01 Gew.-% bis 0,1 Gew.-%, jeweils bezogen auf gesamtes Mittel, eingesetzt werden.

Als in den Mitteln verwendbare Enzyme kommen solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind gewünschtenfalls in den Mitteln in Mengen vorzugsweise nicht über 5 Gew.-%, insbesondere von 0,2 Gew.-% bis 2 Gew.-%, enthalten.

Die Mittel können als optische Aufheller z.B. Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls oder 4-(4-Chlorstyryl)-4'-(2-sulfostyryl)-diphenyls. Auch Gemische der vorgenannten Aufheller können verwendet werden.

Zu den geeigneten Schauminhibitoren gehören z.B. Organopolysiloxane und deren Gemische mit mikrofeiner, gegebenenfalls silanierter Kieselsäure sowie Paraffinwachse und deren Gemische mit silanierter Kieselsäure oder Bisfettsäurealkylendiamiden. Mit Vorteilen werden auch Gemische aus verschiedenen Schaum-inhibitoren verwendet, zum Beispiel solche aus Silikonen, Paraffinen oder Wachsen. Vorzugsweise sind die Schauminhibitoren, insbesondere Silikon- und/oder Paraffinhaltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die Mittel auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem Mittel, das diese öl- und fettlösende Komponente enthält, gewaschen wurde. Zu den bevorzugten öl- und fettlösenden Komponenten zählen z.B. nicht-ionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die Mittel können auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung Polymere aus Vinylpyrrolidon, Vinylimidazol, Vinylpyridin-N-Oxid oder Copolymere aus diesen sind. Brauchbar sind sowohl Polyvinylpyrrolidone mit Molgewichten von 15 000 bis 50 000 wie auch Polyvinylpyrrolidone mit Molgewichten über 1 000 000, insbesondere von 1 500 000 bis 4 000 000, N-Vinylimidazol/N-Vinylpyrrolidon-Copolymere, Polyvinyloxazolidone, Copolymere auf Basis von Vinylmonomeren und Carbonsäureamiden, pyrrolidongruppenhaltige Polyester und Polyamide, gepfropfte Polyamidoamine und Polyethylenimine, Polymere mit Amidgruppen aus sekundären Aminen, Polyamin-N-Oxid-Polymere, Polyvinylalkohole und Copolymere auf Basis von Acrylamidoalkenylsulfonsäuren. Eingesetzt werden können aber auch enzymatische Systeme, umfassend eine Peroxidase und Wasserstoffperoxid beziehungsweise eine in Wasser Wasserstoffperoxid-liefernde Substanz. Der Zusatz einer Mediatorverbindung für die Peroxidase, zum Beispiel eines Acetosyringons, eines Phenolderivats oder eines Phenotiäzins oder Phenoxazins, ist in diesem Fall bevorzugt, wobei auch zusätzlich obengenannte polymere Farbübertragungsinhibitorwirkstoffe eingesetzt werden können. Polyvinylpyrrolidon weist zum Einsatz in erfindungsgemäßen Mitteln vorzugsweise eine durchschnittliche Molmasse im Bereich von 10 000 bis 60 000, insbesondere im Bereich von 25 000 bis 50 000 auf. Unter den Copolymeren sind solche aus Vinylpyrrolidon und Vinylimidazol im Molverhältnis 5:1 bis 1:1 mit einer durchschnittlichen Molmasse im Bereich von 5 000 bis 50 000, insbesondere 10 000 bis 20 000 bevorzugt.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, z.B. -Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, z.B. in Mengen von 0,1 bis 5 Gew.-%, bezogen auf die Mittel, eingesetzt.

Zu den in den Mitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den Mitteln vorzugsweise in Mengen von nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den Mitteln vorzugsweise nicht über 20 Gew.-%, insbesondere von 1,2 Gew.-% bis 17 Gew.-%, enthalten.

Die Herstellung fester Mittel bereitet keine Schwierigkeiten und kann im Prinzip bekannter Weise, zum Beispiel durch Sprühtrocknen oder Granulation, erfolgen, wobei optionale Persauerstoffverbindung und optionaler Bleichkatalysator gegebenenfalls später zugesetzt werden.

Zur Herstellung von Mitteln mit erhöhtem Schüttgewicht, insbesondere im Bereich von 650 g/l bis 950 g/l, ist ein einen Extrusionsschritt aufweisendes Verfahren bevorzugt. Die Herstellung flüssiger Mittel. bereitet ebenfalls keine Schwierigkeiten und kann ebenfalls in bekannter Weise erfolgen.

Gemäß einer Ausführungsform kann die Lehre dazu eingesetzt werden, den Parfümanteil in Wasch-, Reinigungs- und Körperpflegemitteln signifikant herabzusetzen. Dadurch ist es möglich, parfümierte Produkte auch für solche besonders empfindlichen Konsumenten anzubieten, die normal parfümierte Proukte aufgrund spezieller Unverträglichkeiten und Irritationen nur eingeschränkt oder gar nicht verwenden können. In diesem Zusammenhang sind vor allem Hautpflegeprodukte und Deodorantien, aber auch Waschmittel, wie z.B. Handwaschmittel zu nennen.

Ein festes, insbesondere pulverförmiges Waschmittel, das erfindungsgemäß verwendet wird kann neben dem 2-Amino-1,3-propandiol und/oder substituiertes 2-Amino-1,3-propandiol insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5-30 Gew.-%
- Nichtionische Tenside, wie vorzugsweise Fettalkoholpolyglycolether, Alkylpolyglucosid, Fettsäureglucamid z.B. in Mengen von vorzugsweise 0,5-15 Gew.-%
- Gerüststoffe, wie z.B. Zeolith, Polycarboxylat, Natriumcitrat, in Mengen von z.B. 0-70 Gew.-% , vorteilhafterweise 5-60 Gew.-%, vorzugsweise 10-55 Gew.-%, insbesondere 15-40 Gew.-%,
- Alkalien, wie z.B. Natriumcarbonat, in Mengen von z.B. 0-35 Ges:-% vorteilhafterweise 1-30 Gew.-%, vorzugsweise 2-25 Gew.-%, insbesondere 5-20 Gew.-%,
- Bleichmittel, wie z.B. Natriumperborat, Natriumpercarbonat, in Mengen von z.B. 0-30 Gew.-% vorteilhafterweise 5-25 Gew.-%, vorzugsweise 10-20 Gew.-%,
- Korrosionsinhibitoren, z.B. Natriumsilicat, in Mengen von z.B. 0-10 Gew.-%, vorteilhafterweise 1-6 Ges.-%, vorzugsweise 2-5 Gew.-%, insbesondere 3-4 Gew.-%,
- Stabilisatoren, z.B. Phosphonate, vorteilhafterweise 0-1 Gew.-%,
- Schauminhibitor, z.B. Seife, Siliconöle, Paraffine vorteilhafterweise 0-4 Gew.-%, vorzugsweise 0,1-3 Gew.-%, insbesondere 0,2-1 Gew.-%,
- Enzyme, z.B. Proteasen, Amylasen, Cellulasen, Lipasen, vorteilhafterweise 0-2 Gew.-%, vorzugsweise 0,2-1 Gew.-%, insbesondere 0,3-0,8 Gew.-%,
- Vergrauungsinhibitor, z.B. Carboxymethylcellulose, vorteilhafterweise 0-1 Gew.-%,
- Verfärbungsinhibitor, z.B. Polyvinylpyrrolidon-Derivate, vorzugsweise 0-2 Gew.-%,
- Stellmittel, z.B. Natriumsulfat, vorteilhafterweise 0-20 Gew.-%,
- Optische Aufheller, z.B. Stilben-Derivat, Biphenyl-Derivat, vorteilhafterweise 0-0,4 Gew.-%, insbesondere 0,1-0,3 Gew.-%,
- ggf. Duftstoffe
- ggf. Wasser
- ggf. Seife
- ggf. Bleichaktivatoren
- ggf. Cellulosederivate
- ggf. Schmutzabweiser,
Gew.-% jeweils bezogen auf das gesamte Mittel.

In einer Ausführungsform liegt das Wasch-, Reinigungs- oder Pflegemittel in flüssiger Form vor, vorzugsweise in Gelform. Flüssige Wasch-, Reinigungs- oder Pflegemittel haben Wassergehalte Von z.B. 10-95 Gew.-%, vorzugsweise 20-80 Gew.-% und insbesondere 30-70 Gew.-%, bezogen auf das gesamte Mittel. Im Falle von flüssigen Konzentraten kann der Wassergehalt auch besonders gering sein, z.B. < 30 Gew.-%, vorzugsweise < 20 Gew.-%, insbesondere < 15 Gew.-% betragen, Gew.-% jeweils bezogen auf das gesamte Mittel. Die flüssigen Mittel können auch nichtwässrige Lösungsmittel enthalten.

Ein flüssiges, insbesondere gelförmiges Waschmittel kann neben den erfindungsgemäß verwendeten Bestandteilen insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5-40 Gew.-%
- Nichtionische Tenside, wie vorzugsweise Fettalkoholpolyglycolether, Alkylpolyglucosid, Fettsäureglucamid z.B. in Mengen von vorzugsweise 0,5-25 Gew.-%
- Gerüststoffe, wie z.B. Zeolith, Polycarboxylat, Natriumcitrat, vorteilhafterweise 0-15 Gew.-%, vorzugsweise 0,01-10 Gew.-%, insbesondere 0,1-5 Gew.-%,
- Schauminhibitor, z.B. Seife, Siliconöle, Paraffine, in Mengen von z.B. 0-10 Gew.-%, vorteilhafterweise 0,1-4 Gew.-%, vorzugsweise 0,2-2 Gew.-%, insbesondere 1-3 Gew.-%,
- Enzyme, z.B. Proteasen, Amylasen, Cellulasen, Lipasen, in Mengen von z.B. 0-3 Gew.-%, vorteilhafterweise 0,1-2 Gew.-%, vorzugsweise 0,2-1 Gew.-%, insbesondere 0,3-0,8 Gew.-%,
- Optische Aufheller, z.B. Stilben-Derivat, Biphenyl-Derivat, in Mengen von z.B. 0-1 Gew.-%, vorteilhafterweise 0,1-0,3 Gew.-%, insbesondere 0,1-0,4 Gew.-%,
- ggf. Duftstoffe
- ggf. Stabilisatoren,
- Wasser
- ggf. Seife, in Mengen von z.B. 0-25 Gew.-%, vorteilhafterweise 1-20 Gew.-%, vorzugsweise 2-15 Gew.-%, insbesondere 5-10 Gew.-%,
- ggf. Lösungsmittel (vorzugsweise Alkohole), vorteilhafterweise 0-25 Gew.-%, vorzugsweise 1-20 Gew.-%, insbesondere 2-15 Gew.-%, Gew.-% jeweils bezogen auf das gesamte Mittel.

Ein flüssiger Weichspüler kann neben den erfindungsgemäß verwendeten Bestandteilen insbesondere noch Komponenten enthalten, die ausgewählt sind aus den folgenden:
- Kationische Tenside, wie insbesondere Esterquats, z.B. in Mengen von 5-30 Gew.-%,
- Cotenside, wie z.B. Glycerolmonostearat, Stearinsäure, Fettalkohole, Fettalkoholethoxylate, z.B. in Mengen von 0-5 Gew.-%, vorzugsweise 0,1-4 Gew.-%,
- Emulgatoren, wie z.B. Fettaminethoxylate, z.B. in Mengen von 0-4 Gew.-%, vorzugsweise 0,1-3 Gew.-%,
- ggf. Duftstoffe
- Farbstoffe, vorzugsweise im ppm-Bereich
- Stabilisatoren, vorzugsweise im ppm-Bereich
- Lösemittel, wie insbesondere Wasser, in Mengen von vorzugsweise 60-90 Gew.-%,
- Gew.-% jeweils bezogen auf das gesamte Mittel.

### Beispiel:

Ein Versuchsbehälter von 35 I Rauminhalt war mit einer 1 cm großen Öffnung zum Anschluss eines Schlauchs versehen, mit dem eine olfaktorische Kontrolle der darin befindlichen Atmosphäre möglich war. Über eine seitlich Klappe in Bodennähe konnte der Behälter rasch geöffnet und mit Untersuchungsmedien (siehe unten) bestückt werden.

Der Versuch fand bei Raumtemperatur unter Normaldruck statt. Es wurden zwei identisch aufgebaute Behälter eingesetzt. In die gereinigten und geruchsneutralen Behälter wurde eine PetriSchale mit 5 ml einer 10%igen Hexanallösung (in Polyethylenglykol 2000) platziert. Nach einer Stunde wurde die Petri Schale mit dem Hexanal entfernt. Nach 30 min ergab die olfaktorische Kontrolle der beiden Behälter jeweils einen intensiven Geruch nach Hexanal. Mittels einer Sprühflasche wurde eine Prüflösung (siehe unten) fein verteilt in einen der Versuchsbehälter eingebracht. Dabei wurde jeweils ein Sprühstoß mit einem Volumen von 0,1 ml erzeugt. Als Lösungsmittel wurde Wasser verwendet. Die Lösungen hatten einen 10 %igen Aktivstoffgehalt. Unmittelbar nach dem Sprühstoß sowie in den in der Tabelle aufgeführten Zeitabständen wurde eine Beurteilung der Geruchsintensität in der Kammer durchgeführt. Die Skala der Geruchsintensität reicht von stark (Wert 6) bis kaum wahrnehmbar (Wert 1). Die Beurteilung wurde von geruchlich geschulten Menschen vorgenommen.

### Ergebnisse:

| | Intensität nach 0h | Intensität nach 1h | Intensität nach 2h | Intensität nach 4h | Intensität nach 8h |
|---|---|---|---|---|---|
| Kammer mit Serinollösung beaufschlagt | 6 | 4 | 3 | 2 | 1 |
| Kammer ohne Beaufschlagung mit einer Serinollösung | 6 | 6 | 6 | 5 | 4 |
| Kammer mit Methylserinollösung beaufschlage | 6 | 4 | 3 | 2 | 1 |

Die Zugabe von Serinol bzw. Methylserinol in das System ermöglichte also einen Abbau von Fehlgeruch resultierend von Hexanal.

## Patentansprüche

1. Verfahren zum Abbau von Fehlgerüchen, hervorgerufen durch die Anwesenheit von Aldehyden und/oder Ketonen, insbesondere kurzkettigen Aldehyden, **dadurch gekennzeichnet, dass** 2-Amino-1,3-propandiol und/oder substituiertes 2-Amino-1,3-propandiol gemäß der Formel (I) 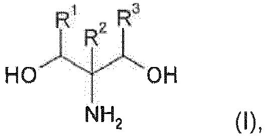 wobei in dieser Formel die Reste R¹, R² sowie R³, jeweils unabhängig voneinander, für Wasserstoff oder Kohlenwasserstoffreste stehen, wobei besonders bevorzugte Reste R² Wasserstoff, Methyl-, Ethyl- und Hydroxymethylreste sind, als Bestandteil eines Wasch- oder Reinigungsmittels zugegeben wird.

2. Verfahren gemäß Anspruch 1, **dadurch gekennzeichnet, dass** das 2-Amino-1,3-propandiol und/oder substituiertes 2-Amino-1,3-propandiol gemäß Formel (I) zusammen mit Riechstoffen eingesetzt wird.

3. Verfahren gemäß einem der Ansprüche 1 oder 2, **dadurch gekennzeichnet, dass** die Zugabe des 2-Amino-1,3-propandiols und/oder des substituierten 2-Amino-1,3-propandiols gemäß Formel (I) durch Sprühen erfolgt.

4. Verfahren gemäß Anspruch 3, **dadurch gekennzeichnet, dass** die Zugabe mittels Spraydose, wie z.B. Druckgasdose, Druckgaspackung, Aerosolpackung, oder mechanisch zu bedienendem Pumpzerstäuber, wie z.B. Pumpspray, erfolgt, unter Bildung eines Sprühnebels, Schaums, Paste oder Flüssigkeitsstrahls.

5. Verfahren gemäß einem der Ansprüche 1 bis 4, **dadurch gekennzeichnet, dass** die Zugabe des 2-Amino-1,3-propandiols und/oder des substituierten 2-Amino-1,3-propan-diols gemäß Formel (I) im Rahmen eines Wasch- oder Reinigungsprozesses erfolgt.

6. Verwendung von 2-Amino-1,3-propandiol und/oder substituiertem 2-Amino-1,3-propandiol gemäß der Formel (I) in, vorzugsweise riechstoffhaltigen, Wasch- oder Reinigungsmitteln, zum Abbau von Fehlgerüchen, hervorgerufen durch die Anwesenheit von Aldehyden und/oder Ketonen, insbesondere kurzkettigen Aldehyden.

## Claims

1. A method for breaking down off-odors caused by the presence of aldehydes and/or ketones, in particular short-chain aldehydes, **characterized in that** 2-amino-1,3-propanediol and/or substituted 2-amino-1,3-propanediol according to formula (I) 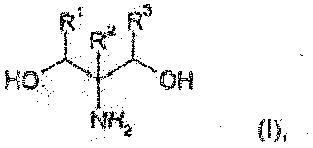 where the R¹, R² and R³ functional groups, each independently of one another, in this formula represent hydrogen or hydrocarbon functional groups, where particularly preferably the R² functional groups are hydrogen, methyl functional groups, ethyl functional groups and hydroxymethyl functional groups, is added as a component of a washing or cleaning agent.

2. The method according to claim 1, **characterized in that** the 2-amino-1,3-propanediol and/or substituted 2-amino-1,3-propanediol according to formula (I) is used together with odorants.

3. The method according to one of claims 1 or 2, **characterized in that** the 2-amino-1,3-propanediol and/or the substituted 2-amino-1,3-propanediol according to formula (I) is added by spraying.

4. The method according to claim 3, **characterized in that** the addition is made by means of a spray can, for example a pressurized gas can, pressurized gas packaging, aerosol packaging, or a mechanically operated pump atomizer, for example a pump spray, to form a spray mist, foam, paste or stream of liquid.

5. The method according to one of claims 1 to 4, **characterized in that** the 2-amino-1,3-propanediol and/or the substituted 2-amino-1,3-propanediol according to formula (I) is added as part of a washing or cleaning process.

6. The use of 2-amino-1,3-propanediol and/or substituted 2-amino-1,3-propanediol according to formula (I) in preferably odorant-containing washing or cleaning agents for breaking down off-odors caused by the presence of aldehydes and/or ketones, in particular short-chain aldehydes.

## Revendications

1. Procédé de décomposition des mauvaises odeurs provoquées par la présence d'aldéhydes et/ou de cétones, en particulier d'aldéhydes à courte chaîne, **caractérisé en ce qu'**on ajoute du 2-amino-1,3-propanediol et/ou un 2-amino-1,3-propanediol substitué selon la formule (I) 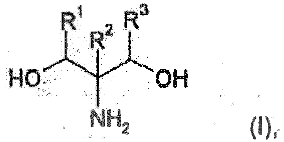 où les résidus R¹, R² ainsi que R³ dans cette formule, respectivement indépendamment les uns des autres, représentent un hydrogène ou des résidus hydrocarbures, où des résidus particulièrement préférentiels R² sont des résidus hydrogène, méthyle, éthyle et hydroxyméthyle, comme composant d'un produit lavant ou nettoyant.

2. Procédé selon la revendication 1, **caractérisé en ce que** le 2-amino-1,3-propanediol et/ou un 2-amino-1,3-propanediol substitué de formule (I) est utilisé ensemble avec des parfums.

3. Procédé selon la revendication 1 ou 2, **caractérisé en ce que** l'ajout du 2-amino-1,3-propanediol et/ou du 2-amino-1,3-propanediol substitué de formule (I) se fait par pulvérisation.

4. Procédé selon la revendication 3, **caractérisé en ce que** l'ajout se fait au moyen d'un pulvérisateur, ex. canette de gaz comprimé, emballage sous pression de gaz, emballage d'aérosol, ou au moyen d'un vaporisateur mécanique, ex. atomiseur à pompe, en formant un brouillard de pulvérisation, une mousse, une pâte ou jet de liquide.

5. Procédé selon l'une quelconque des revendications 1 à 4, **caractérisé en ce que** l'ajout du 2-amino-1,3-propanediol et/ou du 2-amino-1,3-propanediol substitué de formule (I) se fait dans le cadre d'un procédé de lavage ou de nettoyage.

6. Utilisation de 2-amino-1,3-propanediol et/ou de 2-amino-1,3-propanediol substitué de formule (I) dans des produits de lavage ou de nettoyage contenant de préférence des parfums, pour décomposer des mauvaises odeurs provoquées par la présence d'aldéhydes et/ou de cétones, en particulier d'aldéhydes à courte chaîne.
